# EUROPEAN PATENT APPLICATION

(11) **EP 0 714 674 A2**
(43) Date of publication of application: **05.06.1996**
(21) Application number: 96200352.1
(22) Date of filing: 09.09.1991
(51) Int. Cl.: A61M 25/01

(54) **Adjustable catheter contamination shield**

(30) Priority: 21.09.1990 US 586626
(62) Divisional of application: 91919186.6
(71) Applicant: BAXTER INTERNATIONAL INC., Deerfield, IL 60015 (US)
(72) Inventor: Woodgrift, Randal W., Laguna Niguel, CA 92677 (US); Welsh, Gregory P., Newport Beach, CA 92663 (US)
(74) Representative: MacGregor, Gordon

(57) **Abstract**

An adjustable contamination shield (210) includes a distal fitting (212), a proximal fitting (214) and a collapsible shield (216), disposed between said distal (212) and proximal (214) fittings for enclosing an adjustable space (218) therebetween and for preventing contact with a portion of the catheter (22) disposed between the distal (212) and proximal (214) fittings. The shield (216) comprises radially overlapping folds (222,224) when in a collapsed configuration, and controlled expansion thereof is provided when the distal (212) and proximal (214) fittings are moved apart to enable observation through the expanded portion of the collapsible shield (216). Expansion involves the outermost fold, followed by the other folds (222,224) in sequence opening out as the fittings (12,14) are moved apart.

## Description

The present invention generally relates to apparatus for preventing the contamination of a catheter as it is inserted into and withdrawn from a body cavity or lumen. More particularly, the present invention is directed to apparatus including an expandable shield for providing a sterile environment for a catheter enclosed thereby. In view of commonly accepted procedures for the utilization of catheters within a venous lumen, such as balloon-tipped catheters, which include the adjustment of catheter depth, or inserted length, it is most desirable to retain sterility of the exposed catheter portion exterior to the body.

A number of prior art devices have addressed this problem and utilized a protective sheath such as a thin, plastic, flexible and collapsible hollow sleeve, which is disposed exterior to the catheter and supported between distal and proximal fittings. For example, see US-A-4327723 and US-A-4515592.

US-A-4515592 discloses a contamination shield for a catheter, in which a collapsible shield portion is provided between distal and proximal fittings. The collapsible shield portion comprises a flexible tube which is caused to fold concertina fashion when the distal and proximal fittings are relatively moved towards each other.

In these patents the catheter may be inserted through the proximal and distal fittings and a collapsed sleeve before insertion into a venous lumen or the like. The collapsed sleeve may have a length of up to ten centimeters, in view of the fact that it is desirable for it to be expanded to lengths as long as one meter for providing a sterile environment for the catheter. To facilitate the insertion of the catheter through the collapsed sleeve, prior devices as in these patents have provided a length of tube approximately equal to the length of collapsed sleeve and disposed between proximal and distal fittings to provide a passage through the collapsed sleeve and prevent rupture, or puncture of the collapsed sleeve during insertion of the catheter therethrough. Once the distal and proximal fittings of the prior art device are pulled apart, and disconnected from the interconnecting tube, the sleeve is expanded and reinsertion of the catheter through the sleeve is difficult, if not impossible, due to difficulty in mutual alignment of the proximal and distal ends.

This is due to the fact that the prior art sleeves do not expand in a controlled manner in which the sleeve is divided into a totally collapsed portion and a totally expanded portion, the latter enabling visual observation of the catheter therethrough. Rather, in prior art devices the expansion of the sleeve is uncontrolled which results in all of the sleeve being partially expanded between the distal and proximal fittings thus obscuring visual observation of the catheter therethrough.

The present invention provides an adjustable catheter contamination shield as in Claim 1, which enables the alignment of the proximal and distal ends without separate interconnection thereof by a tube or the like.

Claim 1 has a precharacterising part based on US-A-4515592 and a characterising part setting out the distinguishing features of the invention.

An adjustable catheter contamination shield in accordance with the present invention generally includes a distal fitting having a bushing for coupling to introducer and means, defining a bore through the distal fitting, for enabling the passage of the catheter therethrough. A proximal fitting is provided which also includes means, defining a bore therethrough, for enabling passage of the catheter through the proximal fitting.

A collapsible shield portion is disposed between the distal and the proximal fittings which provides means for enclosing an adjustable space therebetween and preventing contact with a portion of the catheter disposed between the distal and the proximal fittings.

The collapsible shield portion comprises radially overlapping folds in the collapsed condition, the outermost fold, followed by the other folds in sequence, opening out as the fittings are moved apart to expand the collapsible shield portion.

### BRIEF DESCRIPTION OF THE DRAWINGS

A better understanding of the present invention may be had by consideration of the following detailed description, taken in conjunction with the accompanying drawings, in which:
Figure 1 is a side view of an adjustable catheter contamination shield, which is not in accordance with the present invention, depicting its use with an introducer for introducing a catheter into a heart;
Figure 2 is an exploded pictorial representation of the contamination shield shown in Figure 1;
Figure 3 is a side view of the shield of Fig. 1, shown in fully collapsed condition;
Figure 4 is a side view of the shield shown in Figure 3 with the catheter inserted therethrough;
Figure 5 is a side view of the shield shown in Figure 3 with a portion of the shield shown in an extended state with the catheter visible therethrough;
Figure 6 is a side view of the shield shown in Figure 3 in a totally expanded condition.
Figure 7 is a cross-sectional view of a portion of a proximal fitting.
Figure 8 is a side view of an alternative proximal fitting;
Figure 9 shows a portion of the proximal fitting bent through approximately 90°;
Figure 10 is view of the fitting of Fig.8;
Figure 11 is a cross-sectional view of an alternative construction of an adjustable catheter contamination shield, in accordance with the present invention; and
Figures 12 and 13 are cross-sectional views of the Figure 11 construction illustrating collapse of the shield.

### DETAILED DESCRIPTION OF THE DRAWINGS

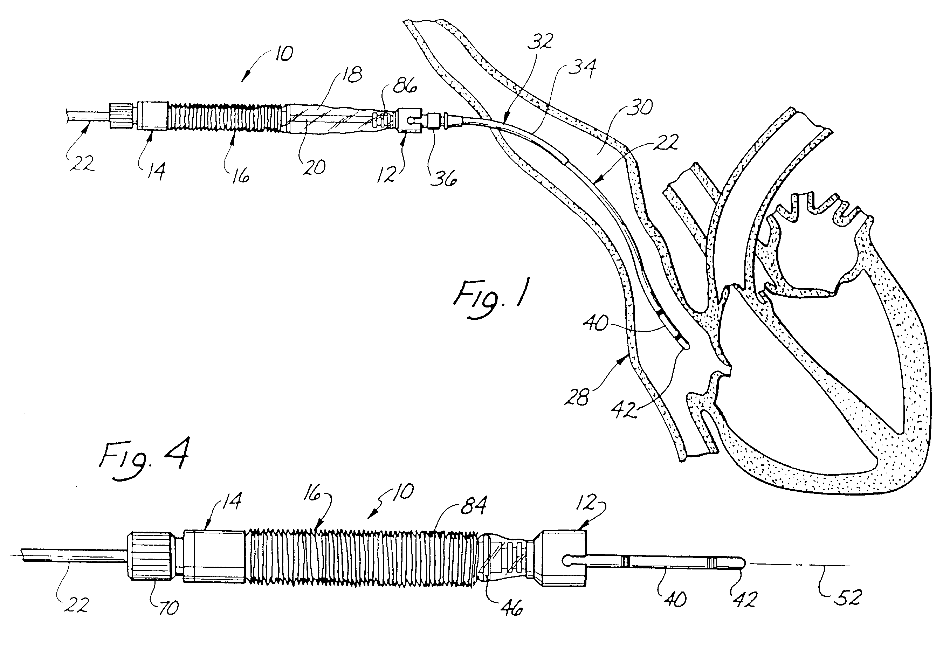

Figure 1 shows an adjustable catheter contamination shield 10 which is not in accordance with the present invention, and generally including a distal fitting 12, a proximal fitting 14, and a collapsible shield 16, disposed between the distal and proximal fittings 14, 12 which provides means for enclosing an adjustable space 18 therebetween and for preventing contact with a portion 20 of a catheter 22 disposed between the distal and proximal fittings 12, 14. As diagrammatically shown in Figure 1, the catheter contamination shield is positioned for the insertion of the catheter 22 into a heart 28 through an internal jugular vein 30 by means of an introducer 32 which includes an introducer sheath 34 and a fitting 36, the introducer 32, including the sheath 34 and fitting 36 being of conventional design. As shown, the catheter 22 may include a balloon portion 40 disposed proximate a tip 42, the balloon portion being commonly used and well-known in the art.

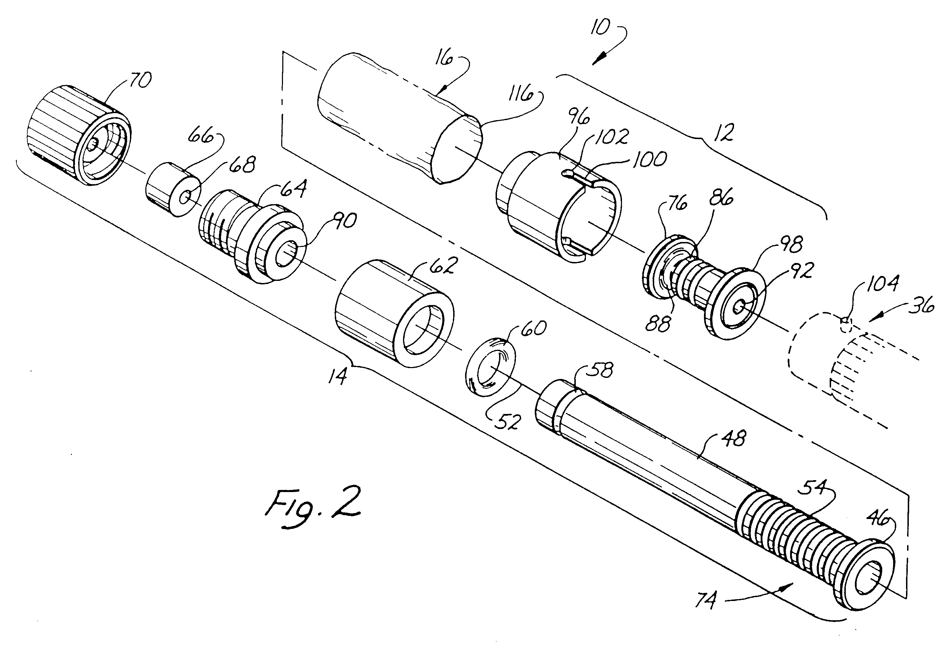

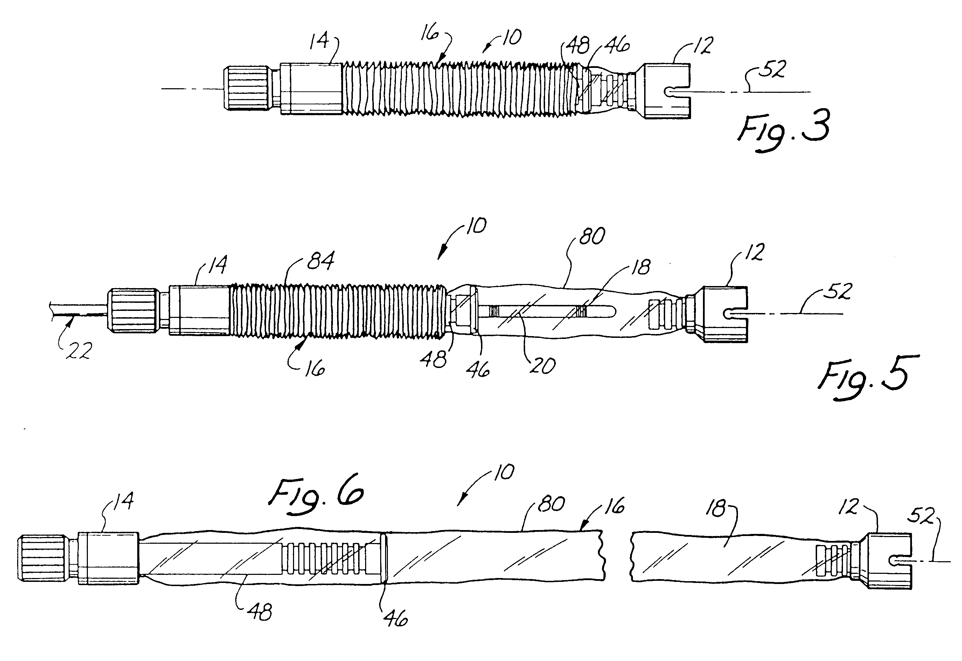

Figure 2 is an exploded perspective view of the catheter contamination shield 10 showing in greater detail the distal fitting 12 and the proximal fitting 14. A flange 46 formed as an integral portion of a tube 48 provides a means for supporting the shield 16 in the collapsed configuration and, in addition, causing the collapsed shield to expand along a longitudinal axis 52 shown in Figures 3 to 6 and hereinafter described in greater detail.

It should be appreciated that, while the flange 46 and tube 48 are shown proximate and connected to the proximal fitting 14, alternatively the flange 46 with tube 48 may be connected with the distal fitting 12, such alternative arrangement not being shown in the drawings. The tube 48, which may be formed from any suitable flexible material and may include a ribbed portion 54 proximate the flange 46, not only provides a passage through the collapsed shield 16 but also importantly provides a means for reducing stress on the catheter passing therethrough which may occur through manual manipulation of the catheter contamination shield 10 and catheter 22 as the latter is advanced through a venous lumen such as the jugular vein 30 as shown in Figure 1.

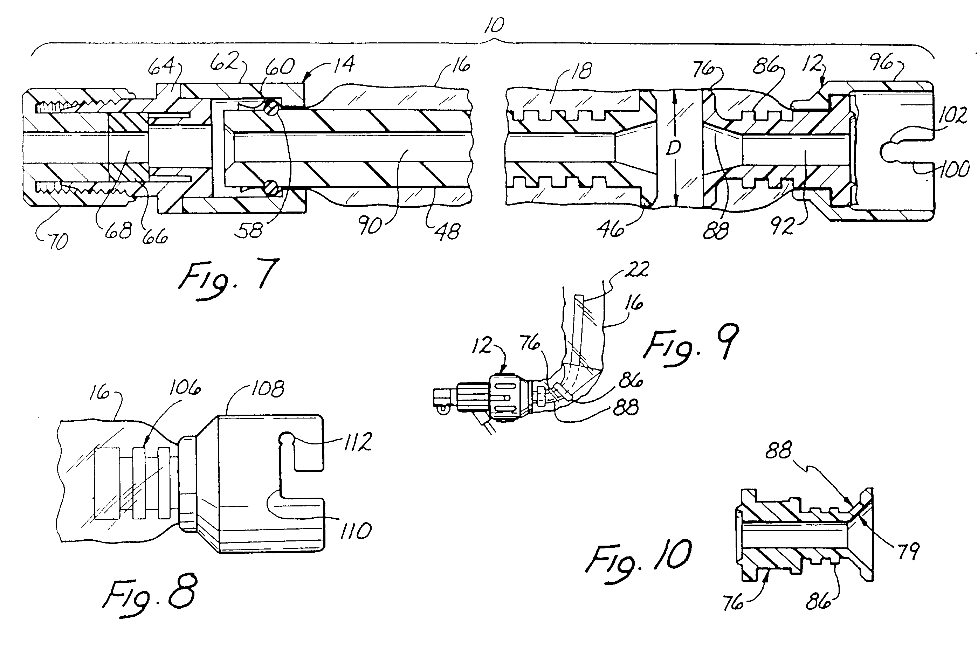

A groove 58 disposed in the flexible tube 48 along with an O-ring 60 and a sleeve 62 provides means for fixing the collapsible shield 16 to the proximal fitting 14 as more clearly shown in cross-section in Figure 7.

Coupled to the sleeve 62 is a threaded fitting 64 enclosing an end bushing 66, having an orifice 68 therethrough sized for fitting into the catheter 22 and held in position by a threaded cap 70 adapted for threaded engagement with the fitting 64 with the bushing 66 there-enclosed, as shown in Figure 7.

As best shown in Figures 5 to 7 the flange 46 has an outside diameter D which is sufficient to prevent the passage of the shield 16 therepast without movement of the distal and proximal fittings 12, 14 from one another. Depending upon the material of construction of the shield 16, this function of the flange 46 in both enabling selected expansion of the shield therepast and for supporting the shield in a collapsed configuration may be obtained when the flange diameter D is greater than the inside diameter of the shield 16. It should be appreciated that while the flange is shown disposed at an end 74, it may be disposed at any intermediate position or a number of flanges, not shown, may be disposed along the tube 48 for controlling the expansion of the shield 16 therepast. The tube 48 provides a clear passage through the collapsed shield 16 and additionally when the shield 16 is collapsed over the tube 48, the flange 46 is coaxially aligned with a flexible distal tube 76 thereby facilitating passage of the catheter 22 therethrough.

The shield 16 may be formed of a polyethylene type material or any other material facilitating the accordion-like collapse thereof over the tube 48 and also, importantly, enabling the visual observation of the catheter 22 through an expanded portion 80 thereof as shown in Figures 5 and 6.

The flange 46 hereinabove described in conjunction with providing a means for maintaining the shield in a selected, collapsed configuration also performs the function of providing tension in the expanded portion 80 between the flange 46 and the distal fitting 12 to enable visual observation of the catheter 22 disposed therein. This state of tension is sufficient to remove a pleating 84 of the shield 16 sufficient to enable the hereinabove visual observation of the catheter. This feature is important in that once the distal and proximal fittings 12, 14 are moved to a spaced apart relationship, the catheter 22 can still be "threaded" or passed from the proximal fitting 14 through the distal fitting 12 by visual alignment thereof through the taut portion 80 of the shield 16.

As shown in Figures 2 and 9, the distal fitting 12 includes a distal tube 76, formed of a flexible material similar to that utilized in the tube 48 and also may include a ribbed portion 86 which provides a means for preventing stress on the catheter 22 during manipulation thereof as hereinabove discussed in connection with the proximal tube 48 and the ribbed portion thereof 54. The threaded fitting 64 and the distal tube 76 include bores 90, 92 for enabling passage of the catheter 22 therethrough. In addition, the distal fitting 12 includes a bushing 96 sized for passing over the tube 76 and sealably engaging a flange 98 disposed on the tube 76 to the introducer 32 by way of the introducer fitting 36. The bushing 96 may include a slot 100 and detent 102 for engaging a prong 104 on the introducer fitting 36 in a conventional manner. Alternatively, as shown in Figure 8, an alternative bushing 108 may include a bayonet-type slot 110 with detent 112 for engaging the prong 104 in a conventional manner.

As shown in Figure 9, the distal fitting 12 can be bent up to approximately 90° without kinking of the catheter therein.

A distal end 116 of the shield 16 may be fixed to the distal fitting 12, for a compressed fit between the flange 98 and the bushing 96.

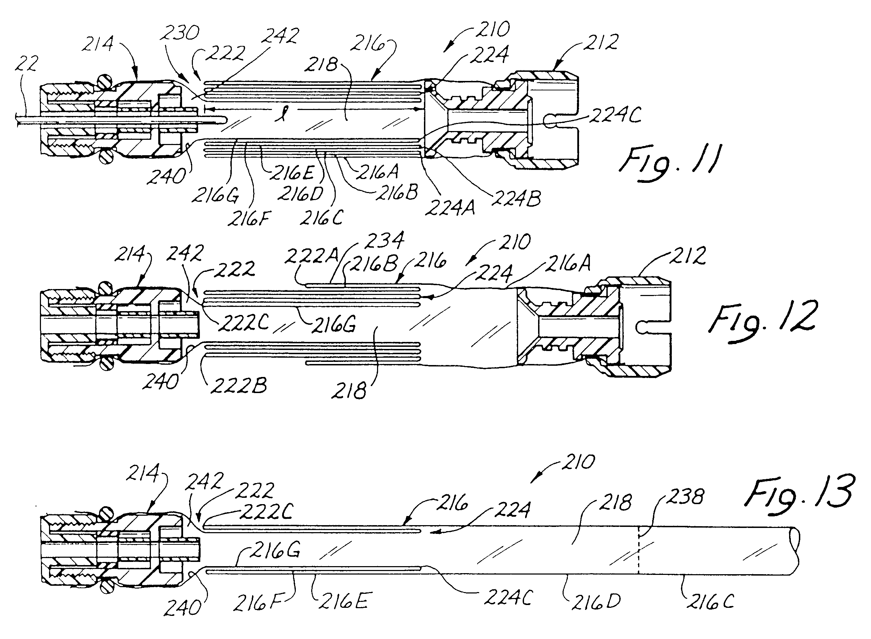

Turning now to Figure 11, there is shown an alternative embodiment 210 in accordance with the present invention generally including a distal fitting 212, a proximal fitting 214, and a collapsible shield 216, disposed between the distal and proximal fittings 212, 214 and enclosing an adjustable space 218 between the fittings. The distal fitting may be identical to the distal fitting 12 hereinabove described and the proximal fitting 214 is similar to the proximal fitting 14 without the tube 48 and flange 46.

The collapsible shield 216 includes a plurality of folds 222, 224, which enables portions 216A-G of the collapsible shield 216 to be disposed in generally parallel relationship with one another when the collapsible shield 216 is in a collapsed configuration as shown in Figure 11, and for enabling the portions 216A-G of the collapsible shield 216 to be disposed in a generally serial relationship with one another when said collapsible shield is in expanded configurations as shown in Figures 12 and 13.

As shown in Figures 11, 12 and 13, the folds are disposed on each end 230, 232 of the collapsible shield portions 216A-G with each of the collapsible shield portions 216A-G having approximately equal length.

As illustrated in Figure 12, when the distal end 212 is pulled away from the proximal fitting 214 the topmost fold 222A moves along a next outside layer 234 of the collapsible shield 216.

As indicated in Figures 12 and 13, the portions 216A-B are arranged in a serial configuration after the distal and proximal fittings have been moved apart from one another, only portions 216C and 216D being shown along with a crease 238 (dashed line) indicating the position of the original fold 222B.

The embodiment 210 of the present invention features structure in which the collapsible shield portions 216A-G are generally tubular between the folds 222, 224 on each end thereof. Because the collapsible shield portions 216A-G are not "bunched", or compacted as in the hereinabove described collapsible shield 16, the expanded configuration as shown in Figure 13 will have greater visibility therethrough without the necessity of providing tension between the distal and proximal fittings 212, 214.

A procedure in accordance with the present invention, and utilizing the structure thereof, provides a sterile environment exterior to a body, such as a heart 28, for the catheter 22 which includes steps of coupling the distal fitting 212 of the contamination shield 210 to an introducer 32, the introducer being adapted for insertion into a venous lumen such as the jugular vein 30 as hereinbefore described.

Thereafter, the procedure in accordance with the present invention includes passing catheter 22 through the proximal fitting 214 and into the sterile area 218 enclosed by the expandable shield 216 disposed between the distal and proximal fittings 212 and 214.

Thereafter, the shield 216 is expanded to enable visual observation of the catheter portion 20 passing between the distal and proximal fittings 212, 214.

## Claims

1. An adjustable catheter contamination shield comprising a distal fitting (212) including a bushing (96) for coupling to an introducer (32) and having a bore for enabling passage of a catheter (22) therethrough; a proximal fitting (214) having a bore therethrough, for enabling passage of the catheter (22) through the proximal fitting; a collapsible shield portion (216), disposed between said distal and proximal fittings (212,214), for enclosing an adjustable space (218) therebetween and for preventing contact with a portion of the catheter disposed between the distal and proximal fittings;
characterised in that the shield portion (216) comprises radially overlapping folds (222,224) in the collapsed condition, the outermost fold, followed by the other folds in sequence opening out as the fittings are moved apart to expand the collapsible shield portion.

2. A shield according to Claim 1, wherein the folds (222,224) are all of equal length.

3. A shield according to Claim 1 or 2, wherein the distal fitting (212,12) includes a flexible tube (76) enabling passage of the catheter (22) therethrough and preventing stress on the catheter.

4. A shield according to Claim 1, 2 or 3, wherein the proximal fitting (214,14) includes a flexible tube (48) enabling passage of the catheter (22) therethrough and preventing stress on the catheter.

5. A shield according to any preceding claim wherein said shield portion (216) is formed of a material enabling visual observation of the catheter through said expanded portion of the shield portion, when the catheter is disposed between the distal and proximal fittings (212,214).

6. A procedure for providing a sterile environment exterior to a body for a catheter (22) comprising the steps of:-
coupling the distal fitting (212) of a contamination shield according to any preceding claim to an introducer (32) which is capable of insertion into a venous lumen; passing the catheter through the proximal fitting (214) of the shield into a sterile area (218) enclosed by the collapsible shield portion (216) of the shield, and expanding the shield portion (216) to enable visual observation of a catheter portion passing between the proximal and distal fittings (214,212).
